# EUROPEAN PATENT APPLICATION

(11) **EP 4 769 328 A1**
(43) Date of publication of application: **01.07.2026**
(21) Application number: 24875704.9
(22) Date of filing: 12.12.2024
(51) Int. Cl.: G06V 10/44, G06V 10/82, G06V 40/16, A61B 5/18, G06V 20/59

(54) **OPTIMIZED DRIVER FATIGUE MONITORING METHOD AND APPARATUS**

(30) Priority: 13.11.2024 CN 202411619139
(71) Applicant: Truly Semiconductors Ltd., Shanwei City, Guangdong 516600 (CN)
(72) Inventor: WANG, Zhixi, Shanwei, Guangdong 516600 (CN); LI, Zhicheng, Shanwei, Guangdong 516600 (CN); ZHENG, Ding, Shanwei, Guangdong 516600 (CN); LI, Xinkai, Shanwei, Guangdong 516600 (CN); WANG, Dezhao, Shanwei, Guangdong 516600 (CN)
(74) Representative: Santarelli
(86) International application number: PCT/CN2024/138653
(87) International publication number: WO 2026/102844

(57) **Abstract**

The present invention discloses an optimized driving fatigue monitoring method. On the basis of performing feature extraction on a driving image using face detection, face position prediction is introduced, and whether to perform feature extraction on the driving image using face detection or to perform feature extraction on the driving image using face prediction is determined according to predicted confidence of a face predicted position. Only when the predicted confidence of the face predicted position does not reach a confidence threshold, the feature extraction is performed on the driving image using face prediction, thereby reducing the number of times to perform feature extraction on the driving image using face prediction and saving computility and time. The present invention further discloses a device for implementing the above driving fatigue monitoring method.

## Description

### Technical Field

The present invention relates to safe driving technologies, in particular to an optimized driving fatigue monitoring method and device.

### Background of the Invention

Fatigue driving is one of the important causes of traffic accidents. Because fatigue driving cannot be strictly prohibited by traffic regulations like drunken driving or driving while making phone calls, fatigue driving is easily overlooked by people. However, when drivers are in a state of fatigue, their attention may be distracted, their strain capacity may decrease, their observation of road conditions and surrounding environments may not be timely, and the drivers may even sleep for a short of time and completely lose their driving ability, which is prone to causing traffic accidents.

In the "dangerous factors affecting the occurrence of traffic accidents" counted in the *World Report on Road Traffic Injury Prevention,* excessive high or improper driving speed, drunken driving, and fatigue driving are main reasons for the occurrence of vehicle accidents, death and severe injuries of all vehicle occupants. According to statistics from China's transport department, traffic accidents caused by fatigue driving account for about 20% of total traffic accidents, 40% of major traffic accidents, and 83% of traffic fatalities.

The China-New Car Assessment Program (C-NCAP) Management Rules (2024 Edition) will be officially implemented from July 1, 2024, with the addition of a driver monitoring system (DMS) evaluation item in an active safety section. Article 6 of Regulation 2019/2144 of the European Union (EU) requires M and N motor vehicles to be equipped with advanced vehicle systems, including a driver drowsiness and attention warning (DDAW) system. The DDAW system should be able to monitor drowsiness of the drivers and issue warnings to the drivers through a human-machine interface (HMI), and privacy and data protection processes of the DDAW system should comply with the requirements of the general data protection regulation (GDPR). The DDAW regulation was officially promulgated and came into effect on August 2021, and the regulation will be mandatory for all new vehicle models from July 2022 and for all new vehicles from July 2024.

The visual-based DMS system adopts a direct monitoring and non-contact solution to characterize fatigue levels of the drivers by collecting image signals of facial, eye, and head movements of the drivers. Compared with indirect monitoring based on a vehicle behavior feature, direct monitoring has higher accuracy and real-time performance, and can detect the state of fatigue faster. For example, the Chinese patent with an application number CN202410210718.7 discloses a driving fatigue detection method based on lightweight neural network image enhancement, including: collecting a dynamic image of a driver in a cockpit in real time, and performing enhancement processing on the dynamic image; capturing a facial image stream subjected to enhancement processing of the driver in the dynamic image through a lightweight and fast facial detector; inputting the facial image stream into a preset facial key feature point extraction network model, and extracting a facial key feature point for each frame of image of the facial image stream, wherein the facial key feature point extraction network model is obtained by constructing and training a reparameterized lightweight convolutional network; and judging, based on the facial key feature points, whether the driver has a facial fatigue feature, and determining that the driver is in fatigue driving when the facial fatigue feature is detected.

However, face detection requires processing the entire dynamic image, and the whole process needs to consume a lot of computility resources and consumes a long time for computation, which seriously affects the timeliness and safety of driving fatigue monitoring.

### Summary of the Invention

In order to overcome shortcomings of the prior art, the present invention provides an optimized driving fatigue monitoring method and device that can reduce the number of times of face detection to save computility and time.

The technical problem to be solved by the present invention is implemented through the following technical solutions:
An optimized driving fatigue monitoring method includes the following steps:
step 100: acquiring an N^{th} driving image, where N≥1;
step 200: judging whether a face predicted position for the N^{th} driving image exists, performing step 300 to step 700 if the face predicted position exists, and performing step 500 to step 700 if no face predicted position exists, wherein the face predicted position for the N^{th} driving image is acquired by predicting according to an (N-1)^{th} driving image;
step 300: acquiring first feature information by performing feature extraction on the N^{th} driving image according to the face predicted position;
step 400: calculating predicted confidence according to the first feature information, judging whether the predicted confidence reaches a confidence threshold, performing step 500 to step 700 if the predicted confidence does not reach the confidence threshold, and taking the first feature information as facial feature information and performing step 700 if the predicted confidence reaches the confidence threshold;
step 500: acquiring a face detected position by performing face detection on the N^{th} driving image;
step 600: acquiring second feature information by performing feature extraction on the N^{th} driving image according to the face detected position, and taking the second feature information as facial feature information; and
step 700: judging whether a driver is in fatigue driving according to the facial feature information.

1. An optimized driving fatigue monitoring method is characterized by including the following steps:
   step 100: acquiring an N^{th} driving image, where N≥1;
   step 200: judging whether a face predicted position for the N^{th} driving image exists, performing step 300 to step 700 if the face predicted position exists, and performing step 500 to step 700 if no face predicted position exists, wherein the face predicted position for the N^{th} driving image is acquired by predicting according to an (N-1)^{th} driving image;
   step 300: acquiring first feature information by performing feature extraction on the N^{th} driving image according to the face predicted position;
   step 400: calculating predicted confidence according to the first feature information, judging whether the predicted confidence reaches a confidence threshold, performing step 500 to step 700 if the predicted confidence does not reach the confidence threshold, and taking the first feature information as facial feature information and performing step 700 if the predicted confidence reaches the confidence threshold;
   step 500: acquiring a face detected position by performing face detection on the N^{th} driving image;
   step 600: acquiring second feature information by performing feature extraction on the N^{th} driving image according to the face detected position, and taking the second feature information as facial feature information; and
   step 700: judging whether a driver is in fatigue driving according to the facial feature information.

Further, in step 300, the step of performing feature extraction on the N^{th} driving image according to the face predicted position is as follows:
step 310: acquiring a first facial image by performing face cropping on the N^{th} driving image according to the face predicted position; and
step 320: acquiring the first feature information by performing feature extraction on the first facial image.

Further, in step 310, when performing face cropping on the N^{th} driving image, the image is cropped by setting a corresponding face recognition box in the N^{th} driving image according to predicted coordinate information of a feature point in the N^{th} image and length and width dimensions of a face in the (N-1)^{th} driving image.

Further, the face predicted position includes coordinate information of at least one feature point in the N^{th} driving image, and the coordinate information is calculated using a position prediction model according to coordinate information of the same feature point in the (N-1)^{th} driving image and a time difference between the two driving images.

Further, in step 600, the step of performing feature extraction on the N^{th} driving image according to the face detected position is as follows:
step 610: acquiring a second facial image by performing face cropping on the N^{th} driving image according to the face detected position; and
step 620: acquiring the second feature information by performing feature extraction on the second facial image.

Further, a feature extraction network is used to perform feature extraction on a first facial image and a second facial image, a backbone network of the feature extraction network adopts an improved mobilenet network combined with a DenseNet network, at the same time, some modules are modified into lighter ghost modules, and accuracy is improved by adding a convolutional layer.

Further, in step 700, a fatigue degree of the driver is calculated according to a proportion of time at which eyes are closed per unit time, the facial feature information includes an eyelid edge feature point and a pupil edge feature point, a proportion of an area where pupils are covered by eyelids is calculated according to coordinate information of the eyelid edge feature point and the pupil edge feature point, when the proportion of the area reaches a first proportion threshold, it is judged that the eyes are in a closed state, a proportion of time when the eyes are in the closed state per unit time is counted, and when the proportion of time reaches a second proportion threshold, it is judged that the driver is in fatigue driving.

Further, after step 700, the method further includes the following steps:
step 800: obtaining a face predicted position of an (N+1)^{th} driving image by performing position prediction on the (N+1)^{th} driving image according to the facial feature information.

An optimized driving fatigue monitoring device is used to implement the driving fatigue monitoring method above, and the driving fatigue monitoring device includes:
an image acquiring module, configured to acquire a driving image;
a position predicting module, configured to acquire a face predicted position by performing face prediction on a next driving image according to a previous driving image;
a position detecting module, configured to acquire a face detected position by performing face detection on the driving image;
a feature extracting module, configured to acquire first feature information or second feature information by performing feature extraction on the driving image according the face predicted position or the face detected position;
a confidence module, configured to determine the first feature information or the second feature information as facial feature information according to predicted confidence of the first feature information; and
a fatigue judging module, configured to judge whether a driver is in fatigue driving according to the facial feature information.

An optimized driving fatigue monitoring device includes a processor and a memory connected with the processor, the memory stores a computer program for execution by the processor, and the processor, when executing the computer program, performs the driving fatigue monitoring method above.

The present invention has the following beneficial effects: the optimized driving fatigue monitoring method of the present invention introduces face position prediction on the basis of performing feature extraction on the driving image using face detection, and whether to perform feature extraction on the driving image using face detection or to perform feature extraction on the driving image using face prediction is determined according to the predicted confidence of the face predicted position. Only when the predicted confidence of the face predicted position does not reach the confidence threshold, the feature extraction is performed on the driving image using face prediction, thereby reducing the number of times to perform feature extraction on the driving image using face prediction and saving computility and time.

### Brief Description of the Drawings

Fig. 1 is a block diagram of steps of a driving fatigue monitoring method provided by the present invention.
Fig. 2 is a schematic architecture diagram of a feature extraction network provided by the present invention.
Fig. 3 is a schematic architecture diagram of a separable convolutional layer in a feature extraction network provided by the present invention.
Fig. 4 is a schematic block diagram of a driving fatigue monitoring device provided by the present invention.
Fig. 5 is a schematic block diagram of another driving fatigue monitoring device provided by the present invention.

### Detailed Description of Embodiments

The present invention is described below in detail with reference to accompanying drawings and embodiments. Examples of the embodiments are shown in the accompanying drawings. The same or similar reference numbers represent the same or similar elements or elements with the same or similar functions from beginning to end. The embodiments described below with reference to the accompanying drawings are only exemplary, are intended to explain the present invention, and cannot be interpreted as a limitation to the present invention.

In the description of the present invention, it should be understood that orientation or position relationships indicated by terms "length", "width", "upper", "lower", "front", "back", "left", "right", "vertical", "horizontal", "top", "bottom", "inside", "outside", etc. are orientation or position relationships shown on the basis of the accompanying drawings, are only for the convenience of describing the present invention and simplifying the description, do not indicate or imply that the device or element referred to must have a specific orientation, and be constructed and operated in a specific orientation, and therefore cannot be understood as a limitation to the present invention.

In addition, terms "first", "second" and "third" are only for the purpose of description, and cannot be understood as indicating or implying relative importance or implicitly indicating the quantity of the indicated technical features. Therefore, the features defined as "first", "second" and "third" may explicitly or implicitly include one or more of the features. In the description of the present invention, unless otherwise limited expressly and specifically, the meaning of "the plurality of" is two or more than two.

In the present invention, unless otherwise expressly stated and limited, the terms such as "install", "connect", "link", "fix" and "arrange" should be understood in a broad sense, for example, they may be fixed connection, or detachable connection, or integration; may be mechanical connection, or electrical connection; and may be direct connection, or indirect connection through an intermediate medium, or internal communication of two elements or interaction between the two elements. Those ordinarily skilled in the art may understand the specific meaning of the above terms in the present invention according to the specific situation.

### Embodiment 1

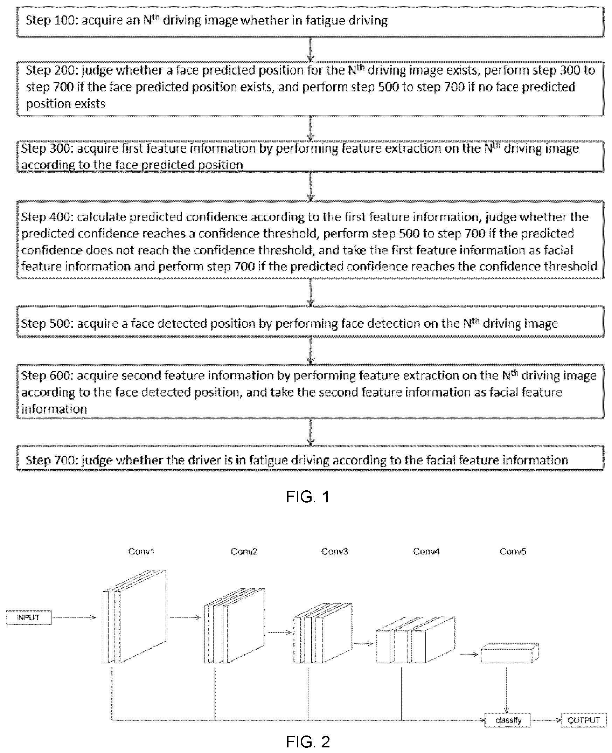

As shown in Fig. 1, an optimized driving fatigue monitoring method includes the following steps:
step 100: an N^{th} driving image is acquired, where N≥1.

In this step 100, a monitoring camera may be set up in front of a driving cab of a vehicle (such as an A-pillar position, and an upper part of a front windshield) and used to photograph a driver in real time in a traveling process of the vehicle, so as to acquire the driving image.

Preferably, the monitoring camera is an infrared camera.

Step 200: whether a face predicted position for the N^{th} driving image exists is judged, step 300 to step 700 are performed if the face predicted position exists, and step 500 to step 700 are performed if no face predicted position exists, wherein the face predicted position for the N^{th} driving image is acquired by predicting according to an (N-1)^{th} driving image.

In this step 200, for a first driving image, since there is no zeroth driving image, a face position of the first driving image naturally cannot be predicted according to the zeroth driving image, that is, a face predicted position of the first driving image does not exist, and thus step 300 to step 700 are performed to calculate a fatigue degree of the first driving image in a face detection manner. For second and subsequent driving images, due to the existence of the first and subsequent driving images, face positions of the second and subsequent driving images naturally can be predicted according to the first and subsequent driving images, and thus step 500 to step 700 are performed to calculate the fatigue degree of the second and subsequent driving images in a face prediction or face detection manner.

Step 300: first feature information is acquired by performing feature extraction on the N^{th} driving image according to the face predicted position.

In this step 300, the face predicted position includes coordinate information of at least one feature point in the N^{th} driving image, and the coordinate information is calculated using a position prediction model according to coordinate information of the same feature point in the (N-1)^{th} driving image and a time difference between the two driving images.

In this embodiment, the feature point used for position prediction includes, but is not limited to an intersection point between a face central axis and a line connecting the two eyes; and the position prediction model may include, but is not limited to a Kalman filter model, a Markov chain model, a particle filter model, etc.

In this step 300, the step of performing feature extraction on the N^{th} driving image according to the face predicted position is as follows:
step 310: a first facial image is acquired by performing face cropping on the N^{th} driving image according to the face predicted position.

In this step 310, since the face predicted position is acquired by predicting according to the (N-1)^{th} driving image, when preforming feature extraction on the (N-1)^{th} driving image, the extracted facial feature information includes coordinate information of the feature point and length and width dimensions of a face. When performing face cropping on the N^{th} driving image, the image may be cropped by setting a corresponding face recognition box in the N^{th} driving image according to the predicted coordinate information of the feature point in the N^{th} image and the length and width dimensions of the face in the (N-1)^{th} driving image.

Step 320: the first feature information is acquired by performing feature extraction on the first facial image.

In this step 320, a feature extraction network may be used to perform feature extraction on the first facial image, and the feature extraction network may be used to perform feature extraction on a second facial image, a backbone network of the feature extraction network adopts an improved mobilenet network combined with DenseNet, at the same time, some modules are modified into lighter ghost modules, and accuracy is improved by adding a convolutional layer.

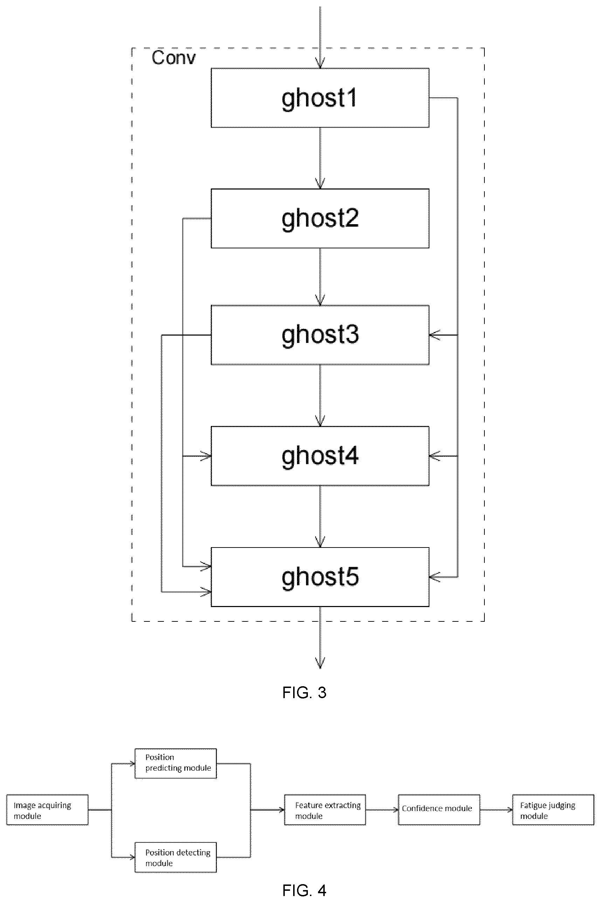

Specifically, as shown in Fig. 2 and Fig. 3, the mobilenet network includes an input layer, a plurality of separable convolutional layers, a classification layer, and an output layer. The input layer, the plurality of separable convolutional layers, the classification layer, and the output layer are connected in sequence, where an output end of each separable convolutional layer is connected to the classification layer except for an input end of the next separable convolutional layer; and each separable convolutional layer includes the plurality of ghost modules, and an input end of each ghost module is connected to output ends of all ghost modules on its upper layer.

Step 400: predicted confidence is calculated according to the first feature information, whether the predicted confidence reaches a confidence threshold is judged, step 500 to step 700 are performed if the predicted confidence does not reach the confidence threshold, and the first feature information is taken as facial feature information and step 700 is performed if the predicted confidence reaches the confidence threshold.

In this step 400, whether there is a significant deviation in the face predicted position acquired by predicting according to the (N-1)^{th} driving image is determined by judging the predicted confidence of the face predicted position. If there is a significant deviation, that is, the predicted confidence does not reach the confidence threshold, step 500 to step 700 are performed, and the fatigue degree of the N^{th} driving image is still calculated in the face detection manner. If there is a small deviation, that is, the predicted confidence reaches the confidence threshold, step 700 is performed, and the fatigue degree of the N^{th} driving image is calculated in the face prediction manner.

The predicted confidence may use a Euclidean distance between a certain feature point in the face predicted position of the N^{th} driving image and the face recognition box. Certainly, the face recognition box may also be calculated using other parameters according to actual needs, and should not be limited to this.

In this embodiment, the feature point used for calculating the predicted confidence includes, but is not limited to the intersection point between the face central axis and the line connecting the two eyes.

Step 500: a face detected position is acquired by performing face detection on the N^{th} driving image.

In this step 500, a face detection network may be used to perform face detection on the N^{th} driving image. The face detection network traverses all pixels in the N^{th} driving image to acquire coordinate information of all the pixels that make up the face in the N^{th} driving image, the face recognition frame, and the length and width dimensions of the face. The whole process requires a large amount of computility resources and consumes a long time for computation.

In this embodiment, the face detection network may be, but is not limited to, a yolo network, a CNN network, an MTCNN network, an RNN network, etc.

Step 600: second feature information is acquired by performing feature extraction on the N^{th} driving image according to the face detected position, and the second feature information is taken as facial feature information.

In this step 600, the face detected position includes the coordinate information of all the pixels that make up the face, the face recognition box, and the length and width dimensions of the face, and is acquired by traversing all pixels in the N^{th} driving image by the face detection network.

In this embodiment, the facial feature information may include, but is not limited to using an intersection point between a face central axis and a line connecting the two eyes as the feature point; and the position prediction model may include, but is not limited to a Kalman filter model, a Markov chain model, a particle filter model, etc.

In this step 600, the step of performing feature extraction on the N^{th} driving image according to the face detected position is as follows:
Step 610: a second facial image is acquired by performing face cropping on the N^{th} driving image according to the face detected position.

In this step 610, the face detected position contains coordinate information of all the feature points and the length and width dimensions of the face. When performing face cropping on the N^{th} driving image, the image may be cropped by setting the corresponding face recognition box in the N^{th} driving image according to the detected coordinate information of all the feature points in the N^{th} image and the length and width dimensions of the face.

Step 620: the second feature information is acquired by performing feature extraction on the second facial image.

In this step 620, a feature extraction network may be used to perform feature extraction on the second facial image, a backbone network of the feature extraction network adopts an improved mobilenet network combined with DenseNet, at the same time, some modules are modified into lighter ghost modules, and accuracy is improved by adding a convolutional layer.

Specifically, as shown in Fig. 2 and Fig. 3, the mobilenet network includes an input layer, a plurality of separable convolutional layers, a classification layer, and an output layer. The input layer, the plurality of separable convolutional layers, the classification layer, and the output layer are connected in sequence, where an output end of each separable convolutional layer is connected to the classification layer except for an input end of the next separable convolutional layer; and each separable convolutional layer includes the plurality of ghost modules, and an input end of each ghost module is connected to output ends of all ghost modules in its upper layer.

Step 700: whether the driver is in fatigue driving is judged according to the facial feature information.

In this step 700, a fatigue degree of the driver is calculated according to a proportion of time at which eyes are closed per unit time, the facial feature information includes an eyelid edge feature point and a pupil edge feature point, a proportion of an area where pupils are covered by eyelids is calculated according to coordinate information of the eyelid edge feature point and the pupil edge feature point, when the proportion of the area reaches a first proportion threshold, it is judged that the eyes are in a closed state, a proportion of time when the eyes are in the closed state per unit time is counted, and when the proportion of time reaches a second proportion threshold, it is judged that the driver is in fatigue driving.

Step 800: a face predicted position of an (N+1)^{th} driving image is obtained by performing position prediction on the (N+1)^{th} driving image according to the facial feature information.

In this step 800, the facial feature information includes coordinate information of at least one feature point in the N^{th} driving image, and the coordinate information of the same feature point in the (N+1)^{th} driving image is predicted using a position prediction model according to coordinate information of the certain feature point in the N^{th} driving image and a time difference between the N^{th} driving image and the (N+1)^{th} driving image.

In this embodiment, the feature point used for position prediction includes, but is not limited to an intersection point between a face central axis and a line connecting the two eyes; and the position prediction model may include, but is not limited to a Kalman filter model, a Markov chain model, a particle filter model, etc.

The optimized driving fatigue monitoring method of the present invention introduces face position prediction on the basis of performing feature extraction on the driving image using face detection, and whether to perform feature extraction on the driving image using face detection or to perform feature extraction on the driving image using face prediction is determined according to the predicted confidence of the face predicted position. Only when the predicted confidence of the face predicted position does not reach the confidence threshold, the feature extraction is performed on the driving image using face prediction, thereby reducing the number of times to perform feature extraction on the driving image using face prediction and saving computility and time.

### Embodiment 2

As shown in Fig. 4, an optimized driving fatigue monitoring device is used to implement the driving fatigue monitoring method described in Embodiment 1, and the driving fatigue monitoring device includes:
an image acquiring module, configured to acquire a driving image;
a position predicting module, configured to acquire a face predicted position by performing face prediction on a next driving image according to a previous driving image;
a position detecting module, configured to acquire a face detected position by performing face detection on the driving image;
a feature extracting module, configured to acquire first feature information or second feature information by performing feature extraction on the driving image according the face predicted position or the face detected position;
a confidence module, configured to determine the first feature information or the second feature information as facial feature information according to predicted confidence of the first feature information; and
a fatigue judging module, configured to judge whether a driver is in fatigue driving according to the facial feature information.

### Embodiment 3

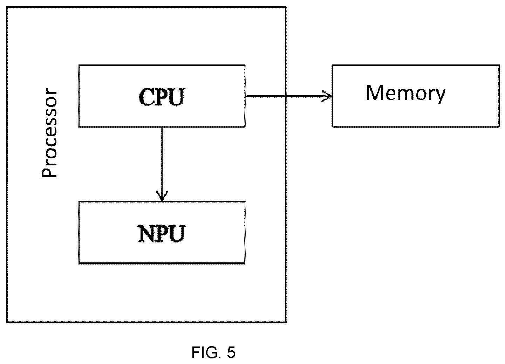

As shown in Fig. 5, an optimized driving fatigue monitoring device includes a processor and a memory connected with the processor, the memory stores a computer program for execution by the processor, and the processor, when executing the computer program, performs the driving fatigue monitoring method described in Embodiment 1.

Finally, it should be noted that the above embodiments are only used to illustrate the technical solutions of the embodiments of the present invention and not to limit them. Although the embodiments of the present invention have been described in detail with reference to the preferred embodiments, those ordinarily skilled in the art should understand that modifications or equivalent substitutions can still be made to the technical solutions of the embodiments of the present invention, and these modifications or equivalent substitutions cannot make the modified technical solutions depart from the scope of the technical solutions of the embodiments of the present invention.

## Claims

1. An optimized driving fatigue monitoring method, **characterized in that**, comprising the following steps:
step 100: acquiring an N^{th} driving image, where N≥1;
step 200: judging whether a face predicted position for the N^{th} driving image exists, performing step 300 to step 700 if the face predicted position exists, and performing step 500 to step 700 if no face predicted position exists, wherein the face predicted position for the N^{th} driving image is acquired by predicting according to an (N-1)^{th} driving image;
step 300: acquiring first feature information by performing feature extraction on the N^{th} driving image according to the face predicted position;
step 400: calculating a predicted confidence according to the first feature information, judging whether the predicted confidence reaches a confidence threshold, performing step 500 to step 700 if the predicted confidence does not reach the confidence threshold, and taking the first feature information as facial feature information and performing step 700 if the predicted confidence reaches the confidence threshold;
step 500: acquiring a face detected position by performing face detection on the N^{th} driving image;
step 600: acquiring second feature information by performing the feature extraction on the N^{th} driving image according to the face detected position, and taking the second feature information as the facial feature information; and
step 700: judging whether a driver is in fatigue driving according to the facial feature information.

2. The driving fatigue monitoring method according to claim 1, **characterized in that**, in step 300, the step of performing the feature extraction on the N^{th} driving image according to the face predicted position is as follows:
step 310: acquiring a first facial image by performing face cropping on the N^{th} driving image according to the face predicted position; and
step 320: acquiring the first feature information by performing the feature extraction on the first facial image.

3. The driving fatigue monitoring method according to claim 2, **characterized in that**, in step 310, when performing the face cropping on the N^{th} driving image, the image is cropped by setting a corresponding face recognition box in the N^{th} driving image according to predicted coordinate information of a feature point in the N^{th} image and length and width dimensions of a face in the (N-1)^{th} driving image.

4. The driving fatigue monitoring method according to any one of claims 1-3, **characterized in that**, the face predicted position comprises coordinate information of at least one feature point in the N^{th} driving image, and the coordinate information is calculated using a position prediction model according to the coordinate information of the same feature point in the (N-1)^{th} driving image and a time difference between the two driving images.

5. The driving fatigue monitoring method according to claim 1, **characterized in that**, in step 600, the step of performing the feature extraction on the N^{th} driving image according to the face detected position is as follows:
step 610: acquiring a second facial image by performing face cropping on the N^{th} driving image according to the face detected position; and
step 620: acquiring the second feature information by performing feature extraction on the second facial image.

6. The driving fatigue monitoring method according to claim 1, **characterized in that**, a feature extraction network is used to perform the feature extraction on a first facial image and a second facial image, a backbone network of the feature extraction network adopts an improved mobilenet network combined with a DenseNet network, at the same time, some modules are modified into lighter ghost modules, and accuracy is improved by adding a convolutional layer.

7. The driving fatigue monitoring method according to claim 1, **characterized in that**, in step 700, a fatigue degree of the driver is calculated according to a proportion of time at which eyes are closed per unit time, the facial feature information comprises an eyelid edge feature point and a pupil edge feature point, a proportion of an area where pupils are covered by eyelids is calculated according to coordinate information of the eyelid edge feature point and the pupil edge feature point, when the proportion of the area reaches a first proportion threshold, it is judged that the eyes are in a closed state, a proportion of time when the eyes are in the closed state per unit time is counted, and when the proportion of time reaches a second proportion threshold, it is judged that the driver is in fatigue driving.

8. The driving fatigue monitoring method according to claim 1, **characterized in that**, after step 700, the method further comprises the following steps:
step 800: obtaining a face predicted position of an (N+1)^{th} driving image by performing position prediction on the (N+1)^{th} driving image according to the facial feature information.

9. An optimized driving fatigue monitoring device, used to implement the driving fatigue monitoring method according to claim 1, **characterized in that**, the driving fatigue monitoring device comprises:
an image acquiring module, configured to acquire the driving image;
a position predicting module, configured to acquire the face predicted position by performing face prediction on a next driving image according to a previous driving image;
a position detecting module, configured to acquire the face detected position by performing the face detection on the driving image;
a feature extracting module, configured to acquire the first feature information or the second feature information by performing the feature extraction on the driving image according the face predicted position or the face detected position;
a confidence module, configured to determine the first feature information or the second feature information as the facial feature information according to predicted confidence of the first feature information; and
a fatigue judging module, configured to judge whether the driver is in fatigue driving according to the facial feature information.

10. An optimized driving fatigue monitoring device, **characterized in that**, comprising a processor and a memory connected with the processor, wherein the memory stores a computer program for execution by the processor, and the processor, when executing the computer program, performs the driving fatigue monitoring method according to claim 1.
